# EUROPEAN PATENT SPECIFICATION

(11) **EP 1 736 123 B1**
(45) Date of publication and mention of the grant of the patent: **01.04.2009**
(21) Application number: 06253286.6
(22) Date of filing: 23.06.2006
(51) Int. Cl.: A61F 5/00, A61B 17/135

(54) **Remote monitoring and adjustment of food intake restriction device**
Fernüberwachung und Steuern einer Vorrichtung zur Begrenzung der Nahrungsaufnahme
Surveillance à distance et ajustement d'un dispositif de restriction de l'injestion de nourriture

(30) Priority: 24.06.2005 US 167861
(43) Date of publication of application: 27.12.2006
(73) Proprietor: ETHICON ENDO-SURGERY, INC., Cincinnati, Ohio 45242 (US)
(72) Inventor: Hassler, Jr., William L., Cincinnati, Ohio 45249 (US)
(74) Representative: Tunstall, Christopher Stephen

(56) References cited:
- EP-A- 0 876 808
- EP-A- 1 442 715
- WO-A-20/04014245
- US-A1- 2001 011 543

## Description

### Field of the Invention

The present invention relates to an implanted restrictive opening device and, more particularly, to a bi-directional communication system for remotely monitoring physiological parameters related to an implanted food intake restriction device and prescribing adjustments for the device from a remote location.

### Background of the Invention

Obesity is becoming a growing concern, particularly in the United States, as the number of obese people continues to increase, and more is learned about the negative health effects of obesity. Morbid obesity, in which a person is 100 pounds or more over ideal body weight, in particular poses significant risks for severe health problems. Accordingly, a great deal of attention is being focused on treating obese patients. One method of treating morbid obesity is to place a restrictive opening device, such as an elongated band, about the upper portion of the stomach. The band is placed so as to form a small gastric pouch above the band and a reduced stoma opening in the stomach. The effect of the band is to reduce the available stomach volume and, thus, the amount of food that can be consumed before becoming "full''. Restrictive gastric bands have typically comprised a fluid-filled elastomeric >balloon with fixed endpoints that encircles the stomach just inferior to the esophago-gastric junction. When fluid is infused into the balloon, the band expands against the stomach, creating the restriction in the stomach. To decrease the restriction in the stomach, fluid is removed from the band.

Restrictive opening devices have also comprised mechanically adjustable bands that similarly encircle the upper portion of the stomach. These bands include any number of resilient materials or gearing devices, as well as drive members, for adjusting the bands. Adjustable bands have also been developed that include both hydraulic and mechanical drive elements. An example of such an adjustable band is disclosed in U.S. Patent No. 6,067,991, entitled "Mechanical Food Intake Restriction Device" which issued on May 30, 2000. It is also known to restrict the available food volume in the stomach cavity by implanting an inflatable elastomeric balloon within the stomach cavity itself. The balloon is filled with a fluid to expand against the stomach wall and, thereby, decrease the available food volume within the stomach.

With each of the above-described types of restrictive opening devices, safe, effective treatment requires that the device be regularly monitored and adjusted to vary the degree of restriction applied to the stomach. With banding devices, the gastric pouch above the band will substantially increase in size following the initial implantation. Accordingly, the stoma opening in the stomach must initially be made large enough to enable the patient to receive adequate nutrition while the stomach adapts to the banding device. As the gastric pouch increases in size, the band is adjusted to vary the stoma size. In addition, it is often desirable to vary the stoma size in order to accommodate changes in the patient's body or treatment regime, or in a more urgent case, to relieve an obstruction or severe esophageal dilatation.

Scheduled physician visits have been required to adjust restrictive opening devices. During these visits, the physician uses a hypodermic needle and syringe to permeate the patient's skin and add or remove saline from the balloon. More recently, implantable pumps have been developed which enable non-invasive adjustments to the band. These pumps are controlled externally by a programmer that communicates with the pump using telemetry command signals. During a scheduled visit, a physician places a hand-held portion of the programmer near the intake restriction implant and transmits power and command signals to the implanted pump. The pump adjusts the fluid levels in the band in response to the commands, and transmits diagnostic data to the programmer.

In addition to adjustments, it is desirable to regularly monitor physiological parameters related to the restrictive opening device to evaluate the efficacy of the treatment. Fluid pressure within the band is of particular importance to monitor to determine the degree of restriction within the patient's stomach. A pressure reading above normal levels may indicate a blockage or infection, while a pressure reading below normal levels may indicate leakage from the balloon. Commonly assigned, co-pending U.S. Patent application number 11/065,410, entitled "Non-invasive Measurement of Fluid Pressure in a Bariatric Device", describes methods for measuring fluid pressure within an intake restriction device to determine the size of the stoma opening. The fluid pressure measurement is communicated to an external programmer placed over the patient's skin in the vicinity of the implant. The pressure measurement from the device can be used to determine the need for an adjustment.

While implanted pumps and pressure measuring systems have greatly enhanced bariatric treatment, a scheduled office visit and one-on-one interaction between the patient and physician has still been necessary to monitor and adjust the device. Oftentimes a great distance separates the physician and patient, necessitating extensive travel for adjustments. The need to schedule an office visit thus increases the complexity of the treatment, and typically results in less monitoring and adjustments than may be desired. Accordingly, it is desirable to provide a method for remotely monitoring the physiological parameters of an implanted restrictive opening device. In addition, it is desirable to provide a bi-directional physician to patient interface that enables a physician to remotely monitor and adjust a restrictive opening device. Through the interface, the physician may evaluate the efficacy of the treatment and prescribe adjustments to be executed by a clinician, or the patient himself, at a different location. The interface enables faster diagnosis of treatment problems, as well as regularly scheduled adjustments such as, for example, to prevent esophageal dilatation or to allow for nightly mucus drainage from the gastric pouch.

EP 1 442 715 A2 discloses a tunable spinal implant and apparatus for its post-operative tuning which enables the implant to be adjusted within a patient.

EP 0 876 808 A1 discloses an adjustable gastric band including a system for adjusting the volume of the gastric band which includes an implantable control box. The control box includes an electrical energy source, an electronic control unit and an electrically driven pump controlled by the unit, for transferring liquid between a reservoir and the gastric band.

US 2001/0011543 A1 discloses a gastric band which is adjusted by an implanted adjustment device. The adjustment device is controlled by a control device in order to reduce or enlarge the size of the stomas opening.

### Summary of the Invention

The present invention provides a bi-directional communication system and method as recited in the claims.

### Brief Description of the Drawings

While the specification concludes with claims particularly pointing out and distinctly claiming the present invention, it is believed the same will be better understood by reference to the following description, taken in conjunction with the accompanying drawings, in which:

FIG. 1 is a simplified, schematic diagram of an implanted restrictive opening device and a bi-directional communication system between the implanted device and a remote monitoring unit;

FIG. 2 is a more detailed, perspective view of an implantable portion of the food intake restriction device shown in FIG.1;

FIG. 3 is a side, partially sectioned view of the injection port shown in FIG. 2;

FIG. 4 is a side, sectional view, taken along line A-A of FIG. 3, illustrating an exemplary pressure sensor for measuring fluid pressure in the intake restriction device of FIG. 2;

FIG. 5 is a simplified schematic of a variable resistance circuit for the pressure sensor shown in FIG. 4;

FIG. 6 is a cross-sectional view of an alternative bi-directional infuser for the food intake restriction device of FIG. 2;

FIG. 7A is a schematic diagram of a mechanically adjustable restriction device incorporating a pressure transducer;

FIG. 7B is a cross-sectional view of the mechanically adjustable device of FIG. 7A taken along line B-B;

FIG. 8 is a block diagram of the major internal and external components of the intake restriction device shown in FIG. 1;

FIG. 9 is a schematic diagram illustrating a number of different communication links between the local and remote units of FIG. 1;

FIG. 10 is a flow diagram of an exemplary communication protocol between the local and remote units for a manually adjustable restriction device;

FIG. 11 is a flow diagram of an exemplary communication protocol between the local and remote units for a remotely adjustable restriction device;

FIG. 12 is a flow diagram of an exemplary communication protocol in which communication is initiated by the patient;

FIG. 13 is a simplified schematic diagram of a data logger for recording pressure measurements from the implanted restriction device;

FIG. 14 is a block diagram illustrating the major components of the data logger shown in FIG. 13; and

FIG. 15 is a graphical representation of a fluid pressure measurement from the sensor shown in FIG. 4, as communicated through the system of the present invention.

Detailed Description of the Invention

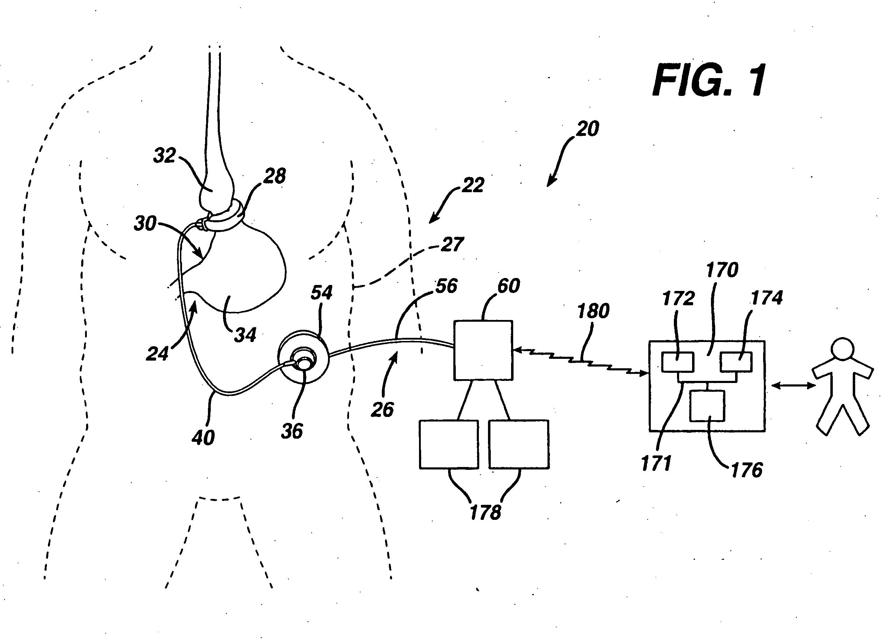

Referring now to the drawings in detail, wherein like numerals indicate the same elements throughout the views, FIG. 1 provides a simplified, schematic diagram of a bi-directional communication system 20 for transmitting data between an implanted restrictive opening device and a remotely located monitoring unit. Through communication system 20, data and command signals may be transmitted between the implanted device and a remotely located physician for monitoring and affecting patient treatment. The communication system of the invention enables a physician to control the restrictive opening device and monitor treatment without meeting face-to-face with the patient. For purposes of the disclosure herein, the terms "remote" and "remotely located" are defined as being at a distance of greater than six feet. In FIG. 1 and the following disclosure, the restrictive opening device is shown and described as being a food intake restriction device 22 for use in bariatric treatment. The use of a food intake restriction device is only representative however, and the present invention may be utilized with other types of implanted restrictive opening devices without departing from the scope of the invention.

As shown in FIG. 1, a first portion 24 of intake restriction device 22 is implanted beneath a patient's skin 27, while a second portion 26 is located external to the patient's skin. Implanted portion 24 comprises an adjustable restriction band 28 that is implanted about the gastrointestinal tract for the treatment of morbid obesity. In this application, adjustable band 28 is looped about the outer wall of a stomach 30 to create a stoma between an upper pouch 32 and a lower pouch 34 of the stomach. Adjustable band 28 may include a cavity made of silicone rubber, or another type of biocompatible material, that inflates inwardly against stomach 30 when filled with a fluid. Alternatively, band 28 may comprise a mechanically adjustable device having a fluid cavity that experiences pressure changes with band adjustments, or a combination hydraulic/mechanical adjustable band.

An injection port 36, which will be described in greater detail below, is implanted in a body region accessible for needle injections and telemetry communication signals. In the embodiment shown, injection port 36 fluidly communicates with adjustable band 28 via a catheter 40. A surgeon may position and permanently implant injection port 36 inside the body of the patient in order to perform adjustments of the food intake restriction or stoma. Injection port 36 is typically implanted in the lateral, subcostal region of the patient's abdomen under the skin and layers of fatty tissue. Alternatively, the surgeon may implant injection port 36 on the sternum of the patient.

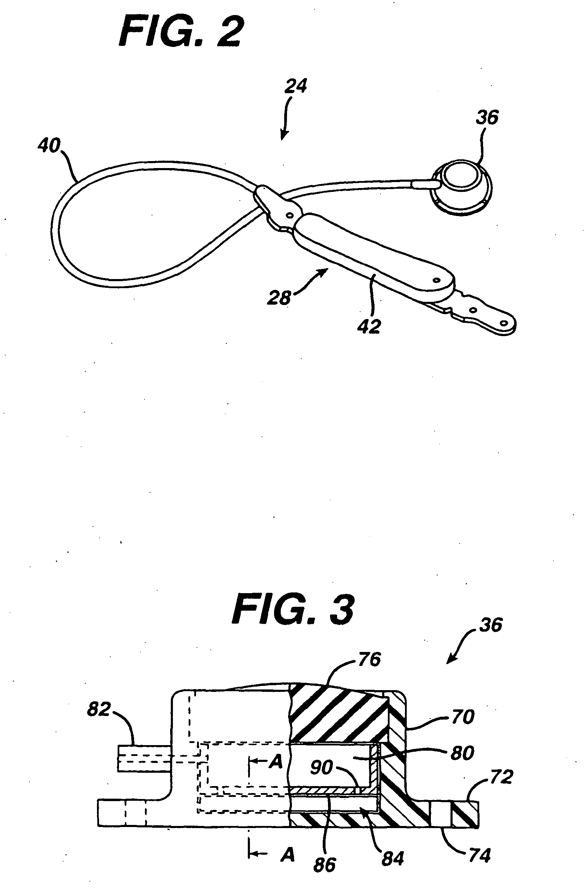

FIG. 2 illustrates adjustable band 28 in greater detail. In this embodiment, band 28 includes a variable volume cavity 42 that expands or contracts against the outer wall of the stomach to form an adjustable stoma for controllably restricting food intake into the stomach. A physician may decrease the size of the stoma opening by adding fluid to variable volume cavity 42 or, alternatively, may increase the stoma size by withdrawing fluid from the cavity. Fluid may be added or withdrawn by inserting a needle into injection port 36. The fluid may be, but is not restricted to, a 0.9 percent saline solution.

Returning now to FIG. 1, external portion 26 of intake restriction device 22 comprises a hand-held antenna 54 electrically connected (in this embodiment via an electrical cable assembly 56) to a local unit 60. Electrical cable assembly 56 may be detachably connected to local unit 60 or antenna 54 to facilitate cleaning, maintenance, usage, and storage of external portion 26. Local unit 60 is a microprocessor-controlled device that communicates with implanted device 22 and a remote unit 170, as will be described further below. Through antenna 54, local unit 60 non-invasively communicates with implanted injection port 36. Antenna 54 may be held against the patient's skin near the location of injection port 36 to transmit telemetry and power signals to injection port 36.

Turning now to FIG. 3, which depicts a side, partially sectioned view of an exemplary injection port 36. As shown in FIG. 3, injection port 36 comprises a rigid housing 70 having an annular flange 72 containing a plurality of attachment holes 74 for fastening the injection port to tissue in a patient. A surgeon may attach injection port 36 to the tissue, such as the fascia covering an abdominal muscle, using any one of numerous surgical fasteners including suture filaments, staples, and clips. Injection port 36 further comprises a septum 76 typically made of a silicone rubber and compressively retained in housing 70. Septum 76 is penetrable by a Huber needle, or a similar type of injection instrument, for adding or withdrawing fluid from the port. Septum 76 self-seals upon withdrawal of the syringe needle to maintain the volume of fluid inside of injection port 36. Injection port 36 further comprises a reservoir 80 for retaining the fluid and a catheter connector 82. Connector 82 attaches to catheter 40, shown in FIG. 2, to form a closed hydraulic circuit between reservoir 80 and cavity 42. Housing 70 and connector 82 may be integrally molded from a biocompatible polymer or constructed from a metal such as titanium or stainless steel.

Injection port 36 also comprises a pressure sensor 84 for measuring fluid pressure within the device. The pressure measured by sensor 84 corresponds to the amount of restriction applied by band 28 to the patient's stomach or other body cavity. The pressure measurement is transmitted from sensor 84 to local unit 60 via telemetry signals using antenna 54. Local unit 60 may display, print and/or transmit the pressure measurement to a remote monitoring unit for evaluation, as will be described in more detail below. In the embodiment shown in FIG. 3, pressure sensor 84 is positioned at the bottom of fluid reservoir 80 within housing 70. A retaining cover 86 extends above pressure sensor 84 to substantially separate the sensor surface from reservoir 80, and protect the sensor from needle penetration. Retaining cover 86 may be made of a ceramic material such as, for example, alumina, which resists needle penetration yet does not interfere with electronic communications between pressure sensor 84 and antenna 54. Retaining cover 86 includes a vent 90 that allows fluid inside of reservoir 80 to flow to and impact upon the surface of pressure sensor 84.

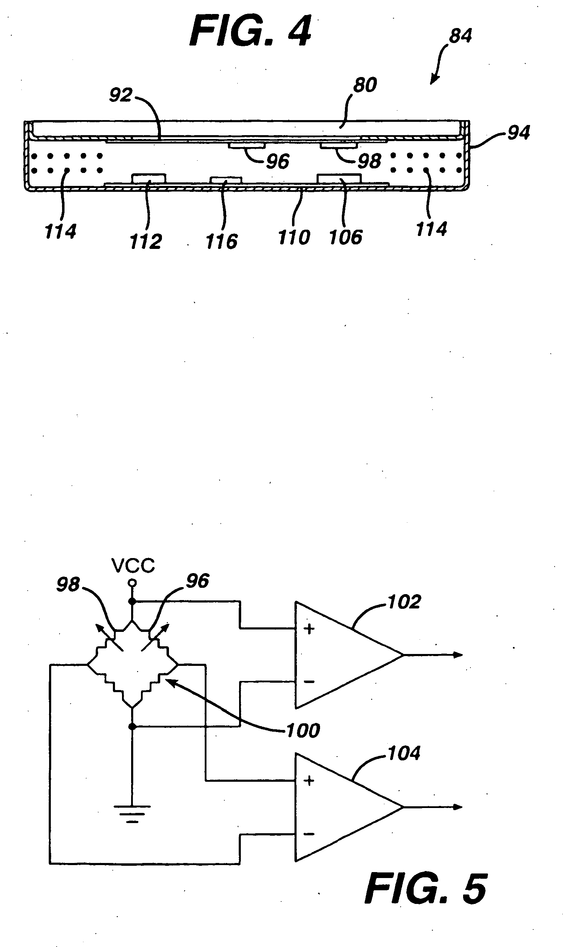

FIG. 4 is a side, sectional view of pressure sensor 84, taken along line A-A of FIG. 3, illustrating an exemplary embodiment for measuring fluid pressure. Pressure sensor 84 is hermetically sealed within a housing 94 to prevent fluid infiltrating and effecting the operation of the sensor. The exterior of pressure sensor 84 includes a diaphragm 92 having a deformable surface. Diaphragm 92 is formed by thinning out a section of the bottom of titanium reservoir 80 to a thickness between 0,025 mm and 0,051 mm (0.001" and 0.002"). As fluid flows through vent 90 in reservoir 80, the fluid impacts upon the surface of diaphragm 92, causing the surface to mechanically displace. The mechanical displacement of diaphragm 92 is converted to an electrical signal by a pair of variable resistance, silicon strain gauges 96, 98. Strain gauges 96, 98 are attached to diaphragm 92 on the side opposite the working fluid in reservoir 80. Strain gauge 96 is attached to a center portion of diaphragm 92 to measure the displacement of the diaphragm. The second, matched strain gauge 98 is attached near the outer edge of diaphragm 92. Strain gauges 96, 98 may be attached to diaphragm 92 by adhesives, or may be diffused into the diaphragm structure. As fluid pressure within band 28 fluctuates, the surface of diaphragm 92 deforms up or down at the bottom of reservoir 80. The deformation of diaphragm 92 produces a resistance change in the center strain gauge 96.

As shown in FIG. 5, strain gauges 96, 98 form the top two resistance elements of a half-compensated, Wheatstone bridge circuit 100. As strain gauge 96 reacts to the mechanical displacements of diaphragm 92, the changing resistance of the gauge changes the potential across the top portion of the bridge circuit. Strain gauge 98 is matched to strain gauge 96 and athermalizes the Wheatstone bridge circuit. Differential amplifiers 102, 104 are connected to bridge circuit 100 to measure the change in potential within the bridge circuit due to the variable resistance strain gauges. In particular, differential amplifier 102 measures the voltage across the entire bridge circuit, while differential amplifier 104 measures the differential voltage across the strain gauge half of bridge circuit 100. The greater the differential between the strain gauge voltages, for a fixed voltage across the bridge, the greater the pressure difference. If desired, a fully compensated Wheatstone bridge circuit could also be used to increase the sensitivity and accuracy of the pressure sensor 84. In a fully compensated bridge circuit, four strain gauges are attached to the surface of diaphragm 92, rather than only two strain gauges as shown in FIG. 4.

Returning to FIG. 4, the output signals from differential amplifiers 102, 104 are applied to a microcontroller 106. Microcontroller 106 is integrated into a circuit board 110 within housing 94. A temperature sensor 112 measures the temperature within injection port 36 and inputs a temperature signal to microcontroller 106. Microcontroller 106 uses the temperature signal from sensor 112 to compensate for variations in body temperature and residual temperature errors not accounted for by strain gauge 98. Compensating the pressure measurement signal for variations in body temperature increases the accuracy of the pressure sensor 84. Additionally, a TET/telemetry coil 114 is located within housing 94. Coil 114 is connected to a capacitor 116 to form a tuned tank circuit for receiving power from and transmitting physiological data, including the measured fluid pressure, to local unit 60. FIGS. 3-5 illustrate one exemplary embodiment for measuring fluid pressure within an intake restriction device. Additional embodiments for measuring fluid pressure are described in U.S. patent application no. 11/065,410 entitled " Non-invasive Measurement of Fluid Pressure in a Bariatric Device".

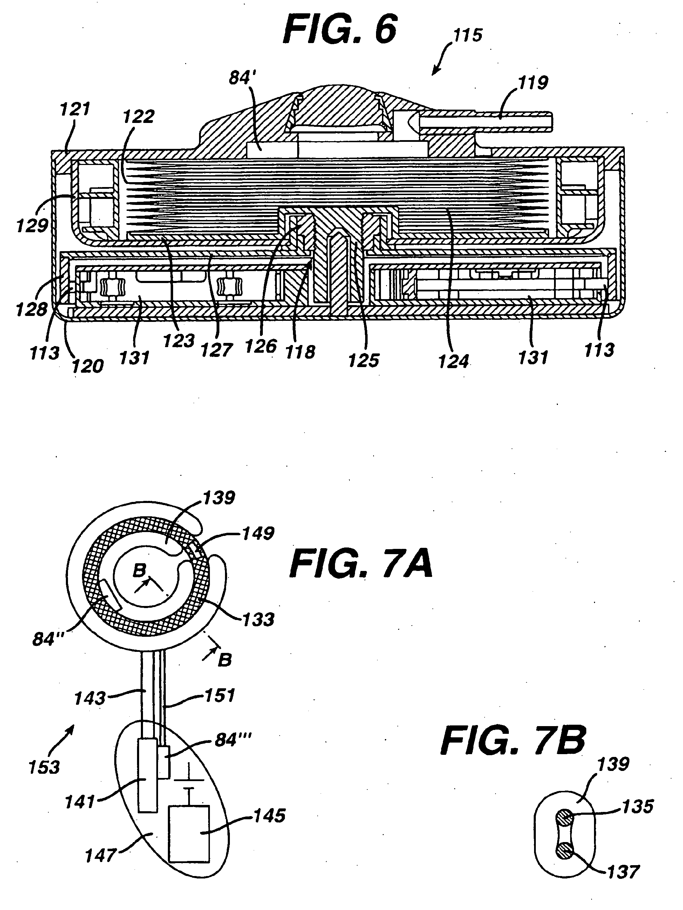

As an alternative to injection port 36, implanted portion 24 may include a bi-directional infuser for varying the fluid level within the adjustable restriction band 28. With an infuser, fluid can be added or withdrawn from band 28 via telemetry command signals, without the need to insert a syringe through the patient's skin and into the port septum. FIG. 6 is a cross-sectional view of an exemplary infuser 115. As shown in FIG. 6, infuser 115 includes a pump, designated generally as 118, for non-invasively transferring fluid into or out of the band in response to telemetry command signals. Pump 118 is encased within a cylindrical outer housing 120 having an annular cover 121 extending across a top portion. A collapsible bellows 122 is securely attached at a top peripheral edge to cover 121. Bellows 122 is comprised of a suitable material, such as titanium, which is capable of repeated flexure at the folds of the bellows, but which is sufficiently rigid so as to be noncompliant to variations in pressure. A lower peripheral edge of bellows 122 is secured to an annular bellows cap 123, which translates vertically within pump 118. The combination of cover 121, bellows 122 and bellows cap 123 defines the volume of a fluid reservoir 124. A catheter connector 119 attaches to catheter 40 (shown in FIG. 2) to form a closed hydraulic circuit between the band and fluid reservoir 124. The volume in reservoir 124 may be expanded by moving bellows cap 123 in a downward direction, away from cover 121. As bellows cap 123 descends, the folds of bellows 122 are stretched, creating a vacuum to pull fluid from the band, through catheter 40 and connector 119, and into reservoir 124. Similarly, the volume in reservoir 124 may be decreased by moving bellows cap 123 in an upward direction towards cover 121, thereby compressing the folds of bellows 122 and forcing fluid from the reservoir through catheter 40 and connector 119 and into band 28.

Bellows cap 123 includes an integrally formed lead screw portion 125 that operatively engages a matching thread on a cylindrical nut 126. The outer circumference of nut 126 is securely attached to an axial bore of a rotary drive plate 127. A cylindrical drive ring 128 is in turn mounted about the outer annular edge of rotary drive plate 127. Nut 126, drive plate 127 and drive ring 128 are all securely attached together by any suitable means to form an assembly that rotates as a unit about an axis formed by screw portion 125. A bushing frame 129 encloses TET and telemetry coils (not shown) for transmitting power and data signals between antenna 54 and pump 118.

Drive ring 128 is rotatably driven by one or more piezoelectric harmonic motors. In the embodiment shown in FIG. 6, two harmonic motors 131 are positioned so that a tip 113 of each motor is in frictional contact with the inner circumference of drive ring 128. When motors 131 are energized, tips 113 vibrate against drive ring 128, producing a "walking" motion along the inner circumference of the ring that rotates the ring. A microcontroller (not shown) in pump 118 is electrically connected to the TET and telemetry coils for receiving power to drive motors 131, as well as receiving and transmitting data signals for the pump. To alter the fluid level in band cavity 42, an adjustment prescription is transmitted by telemetry from antenna 54. The telemetry coil in infuser 115 detects and transmits the prescription signal to the microcontroller. The microcontroller in turn drives motors 131 an appropriate amount to collapse or expand bellows 122 and drive the desired amount of fluid to/from band 28.

In order to measure pressure variations within infuser 115, and, thus, the size of the stoma opening, a pressure sensor, indicated by block 84', is included within bellows 122. Pressure sensor 84' is similar to pressure sensor 84 described above. As the pressure against band 28 varies due to, for example, peristaltic pressure from swallowing, the fluid in band 28 experiences pressure changes. These pressure changes are conveyed back through the fluid in catheter 40 to bellows 122. The diaphragm in pressure sensor 84' deflects in response to the fluid pressure changes within bellows 122. The diaphragm deflections are converted into an electrical signal indicative of the applied pressure in the manner described above with respect to FIGS. 4 and 5. The pressure signal is input to the infuser microcontroller, which transmits the pressure to a monitoring unit external to the patient via the telemetry coil. Additional details regarding the operation of bi-directional infuser 115 may be found in commonly-assigned, co-pending U.S. Patent application number 11/065,410 entitled " Non-invasive Measurement of Fluid Pressure in a Bariatric Device".

FIGS. 7A and 7B depict a mechanically adjustable band 153 for creating a food intake restriction in the abdomen of a patient. Mechanical band 153 may be used as an alternative to hydraulically adjustable band 28 for creating a stoma. Mechanically adjustable band 153 comprises a substantially circular resilient core 133 having overlapping end portions 135, 137. Core 133 is substantially enclosed in a fluid-filled compliant housing 139. A releasable and lockable joint 149 of core 133 protrudes from the ends of housing 139 to enable the core and housing to be placed around the esophagus or stomach of a patient to form a stoma. An implanted motor 141 is spaced from core 133 to mechanically adjust the overlap of the core end portions 135, 137 and, accordingly, the stoma size formed by the core. Motor 141 adjusts the size of core 133 through a drive shaft 143 that is connected to a drive wheel (not shown) within housing 139. Motor 141 is molded together with a remote-controlled power supply unit 145 in a body 147 comprised of silicon rubber, or another similar material.

As motor 141 changes the size of core 133, the pressure of the fluid within housing 139 varies. To measure the pressure variations, a pressure sensor, similar to that described above, is placed in communication with the fluid of housing 139. The pressure sensor may be placed within housing 139, as shown by block 84", so that the pressure variations within the stoma opening are transferred through the fluid in housing 139 to the diaphragm of the sensor. Sensor 84" translates the deflections of the diaphragm into a pressure measurement signal, which is transmitted to an external unit via telemetry in the manner described above. In an alternative scenario, the pressure sensor may be placed within the implanted motor body 147, as indicated by block 84''', and fluidly connected to housing 139 via a tube 151 extending alongside drive shaft 143. As fluid pressure varies in housing 139 due to pressure changes within the stoma opening, the pressure differentials are transferred through the fluid in tube 151 to sensor 84'''. Sensor 84''' generates an electrical signal indicative of the fluid pressure. This signal is transmitted from the patient to an external unit in the manner described above.

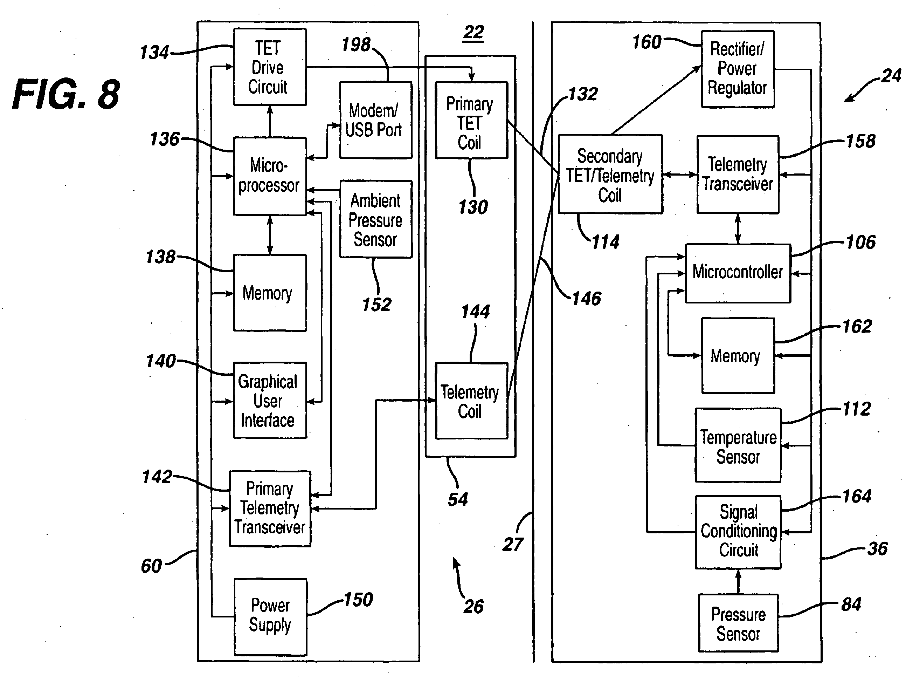

FIG. 8 is a block diagram illustrating the major components of implanted and external portions 24, 26 of intake restriction device 22. As shown in FIG. 8, external portion 26 includes a primary TET coil 130 for transmitting a power signal 132 to implanted portion 24. A telemetry coil 144 is also included for transmitting data signals to implanted portion 24. Primary TET coil 130 and telemetry coil 144 combine to form antenna 54 as shown. Local unit 60 of external portion 26 includes a TET drive circuit 134 for controlling the application of power to primary TET coil 130. TET drive circuit 134 is controlled by a microprocessor 136. A graphical user interface 140 is connected to microprocessor 136 for inputting patient information and displaying and/or printing data and physician instructions. Through user interface 140, the patient or clinician can transmit an adjustment request to the physician and also enter reasons for the request. Additionally, user interface 140 enables the patient to read and respond to instructions from the physician.

Local unit 60 also includes a primary telemetry transceiver 142 for transmitting interrogation commands to and receiving response data, including sensed fluid pressure, from implanted microcontroller 106. Primary transceiver 142 is electrically connected to microprocessor 136 for inputting and receiving command and data signals. Primary transceiver 142 drives telemetry coil 144 to resonate at a selected RF communication frequency. The resonating circuit generates a downlink alternating magnetic field 146 that transmits command data to implanted microcontroller 106. Alternatively, transceiver 142 may receive telemetry signals transmitted from secondary coil 114. The received data may be stored in a memory 138 associated with microprocessor 136. A power supply 150 supplies energy to local unit 60 in order to power intake restriction device 22. An ambient pressure sensor 152 is connected to microprocessor 136. Microprocessor 136 uses the signal from ambient pressure sensor 152 to adjust the received fluid pressure measurement for variations in atmospheric pressure due to, for example, variations in barometric conditions or altitude.

FIG. 8 also illustrates the major components of implanted portion 24 of device 22. As shown in FIG. 8, secondary TET/telemetry coil 114 receives power and communication signals from external antenna 54. Coil 114 forms a tuned tank circuit that is inductively coupled with either primary TET coil 130 to power the implant, or primary telemetry coil 144 to receive and transmit data. A telemetry transceiver 158 controls data exchange with coil 114. Additionally, implanted portion 24 includes a rectifier/power regulator 160, microcontroller 106 described above, a memory 162 associated with the microcontroller, temperature sensor 112, pressure sensor 84 and a signal conditioning circuit 164 for amplifying the signal from the pressure sensor. The implanted components transmit the temperature adjusted pressure measurement from sensor 84 to local unit 60 via antenna 54. The pressure measurement may be stored in memory 138 within local unit 60, shown on a display within local unit 60, or transmitted in real time to a remote monitoring station.

As mentioned hereinabove, it is desirable to provide a communication system for the remote monitoring and control of an intake restriction device. Through the communication system, a physician may retrieve a history of fluid pressure measurements from the restriction device to evaluate the efficacy of the bariatric treatment. Additionally, a physician may downlink instructions for a device adjustment. A remotely located clinician may access the adjustment instructions through local unit 60. Using the instructions, the clinician may inject a syringe into injection port 36 and add or remove saline from fluid reservoir 80 to accomplish the device adjustment. Alternatively, the patient may access the instructions through local unit 60, and non-invasively execute the instructions in infuser 115 or mechanically adjustable band 153 using antenna 54. Real-time pressure measurements may be uplinked to the physician during the adjustment for immediate feedback on the effects of the adjustment. Alternatively, the patient or clinician may uplink pressure measurements to the physician after an adjustment for confirmation and evaluation of the adjustment.

As shown in FIG. 1, communication system 20 includes local unit 60 and a remote monitoring unit 170, also referred to herein as a base unit. Remote unit 170 may be located at a physician's office, hospital or other location convenient to the physician. Remote unit 170 is a personal computer type device comprising a microprocessor 172, which may be, for example, an Intel Pentium® microprocessor or the like. A system bus 171 interconnects microprocessor 172 with a memory 174 for storing data such as, for example, physiological parameters and patient instructions. A graphical user interface 176 is also interconnected to microprocessor 172 for displaying data and inputting instructions and correspondence to the patient. User interface 176 may comprise a video monitor, a touchscreen, or other display device, as well as a keyboard or stylus for entering information into remote unit 170.

A number of peripheral devices 178 may interface directly with local unit 60 for inputting physiological data related to the patient's condition. This physiological data may be stored in local unit 60 and uploaded to remote unit 170 during an interrogation or other data exchange. Examples of peripheral devices that can be utilized with the present invention include a weight scale, blood pressure monitor, thermometer, blood glucose monitor, or any other type of device that could be used outside of a physician's office to provide input regarding the current physiological condition of the patient. A weight scale, for example, can electrically communicate with local unit 60 either directly, or wirelessly through antenna 54, to generate a weight loss record for the patient. The weight loss record can be stored in memory 138 of local unit 60. During a subsequent interrogation by remote unit 170, or automatically at prescheduled intervals, the weight loss record can be uploaded by microprocessor 136 to remote unit 170. The weight loss record may be stored in memory 174 of remote unit 170 until accessed by the physician.

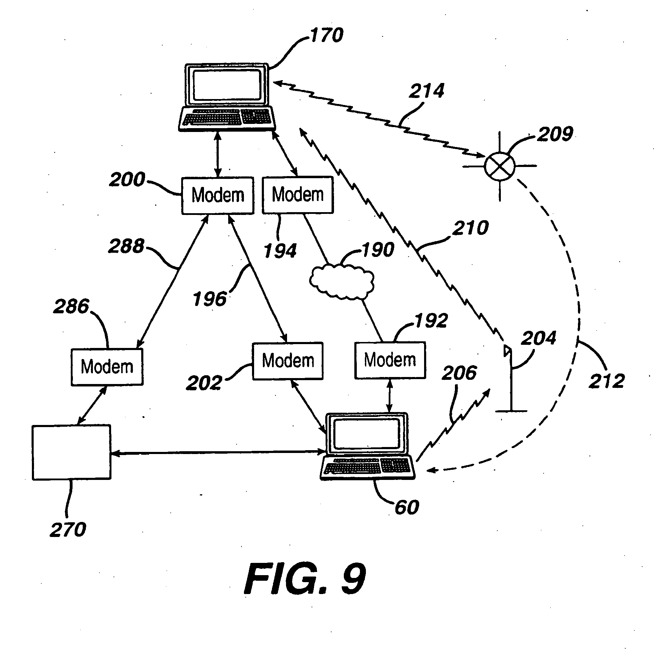

Also as shown in FIG. 1, a communication link 180 is created between local unit 60 and remote unit 170 for transmitting data, including voice, video, instructional information and command signals, between the units. Communication link 180 may comprise any of a broad range of data transmission media including web-based systems utilizing high-speed cable or dial-up connections, public telephone lines, wireless RF networks, satellite, T1 lines or any other type of communication medium suitable for transmitting data between remote locations. FIG. 9 illustrates various media for communication link 180 in greater detail. As shown in FIG. 9, local and remote units 60, 170 may communicate through a number of different direct and wireless connections. In particular, the units may communicate through the Internet 190 using cable or telephone modems 192, 194. In this instance, data may be transmitted through any suitable Internet communication medium such as, for example, e-mail, instant messaging, web pages, or document transmission. Alternatively, local and remote units 60, 170 may be connected through a public telephone network 196 using modems 200, 202. Units 60, 170 may also communicate through a microwave or RF antenna 204 via tunable frequency waves 206, 210. A communication link may also be established via a satellite 209 and tunable frequency waves 212, 214. In addition to the links described above, it is envisioned that other types of transmission media, that are either known in the art or which may be later developed, could also be utilized to provide the desired data communication between local and remote units 60, 170 without departing from the scope of the invention.

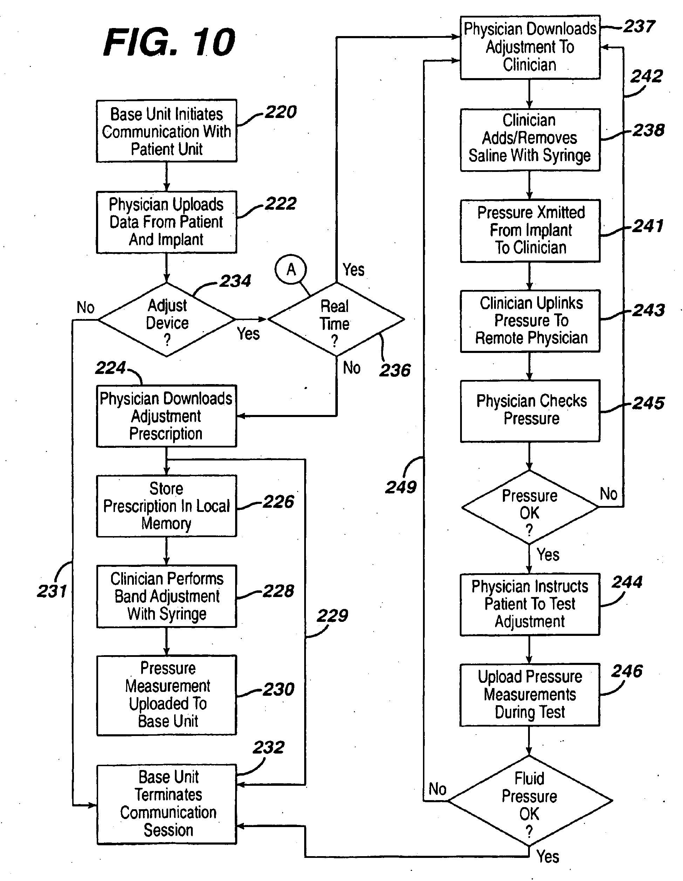

FIG. 10 is a data flow diagram of an exemplary interaction using bi-directional communication system 20. In this interaction, a physician may download an adjustment prescription that is subsequently manually executed by a clinician present with the patient. A physician initiates the communication session between remote unit 170 and local unit 60 as shown at step 220. The session may be initiated by transmitting an e-mail or instant message via the Internet link 190, or through any of the other communication links described with respect to FIG. 9. During the communication session, the physician may download instructions to memory 138, or may upload previously stored data obtained from device 22 or peripheral devices 178, as shown at step 222. This data may include fluid pressure, a weight history, or a patient compliance report. After the data is uploaded, the physician may evaluate the data and determine the need for a device adjustment, as shown at step 234. If an adjustment is indicated, the physician may download an adjustment prescription command to local unit 60 as shown at step 224. Local unit 60 stores the prescription in memory 138 for subsequent action by a clinician, as shown by step 226. With the patient present, the clinician accesses the prescription from memory 138. The clinician then inserts a syringe into septum 76 of injection port 36 and adds or withdraws the fluid volume specified in the prescription. Following the adjustment, the clinician places antenna 54 over the implant and instructs microcontroller 106 to transmit pressure measurements from sensor 84 to local unit 60. The pressure measurements are uploaded by microprocessor 136 in local unit 60 to remote unit 170, as shown at step 230, to provide a confirmation to the physician that the adjustment instructions were executed, and an indication of the resulting effect on the patient. In an off-line adjustment, the base unit terminates communication with local unit 60 following the downloading of the adjustment prescription, as shown by line 229, or following receipt of the patient data if an adjustment is not indicated, as shown by line 231.

In addition to the off-line adjustment session of steps 220-234, a physician may initiate a real-time interactive adjustment, as indicated at step 236, in order to monitor the patient's condition before, during and after the adjustment. In this instance, the physician downloads an adjustment prescription, as shown at step 237, while the patient is present with a clinician. The clinician inserts a syringe into septum 76 of injection port 36 and adds or withdraws the specified fluid from reservoir 80, as shown at step 238, to execute the prescription. After the injection, the physician instructs the clinician to place antenna 54 over the implant, as shown at step 241, to transmit fluid pressure measurements from the implant to local unit 60. The pressure measurements are then uplinked to the physician through link 180, as shown at step 243. The physician evaluates the pressure measurements at step 245. Based upon the evaluation, the physician may provide further instructions through link 180 to readjust the band as indicated by line 242. Additionally, the physician may provide instructions for the patient to take a particular action, such as eating or drinking, to test the adjustment, as shown at step 244. As the patient performs the test, the physician may upload pressure measurements from the implant, as shown at step 246, to evaluate the peristaltic pressure against the band as the food or liquid attempts to pass through the stoma. If the pressure measurements are too high, indicating a possible obstruction, the physician may immediately transmit additional command signals to the clinician to readjust the band and relieve the obstruction, as indicated by line 249. After the physician is satisfied with the results of the adjustment, the communication session is terminated at step 232. As shown in the flow diagram, communication link 180 enables a physician and patient to interact in a virtual treatment session during which the physician can prescribe adjustments and receive real-time fluid pressure feedback to evaluate the efficacy of the treatment.

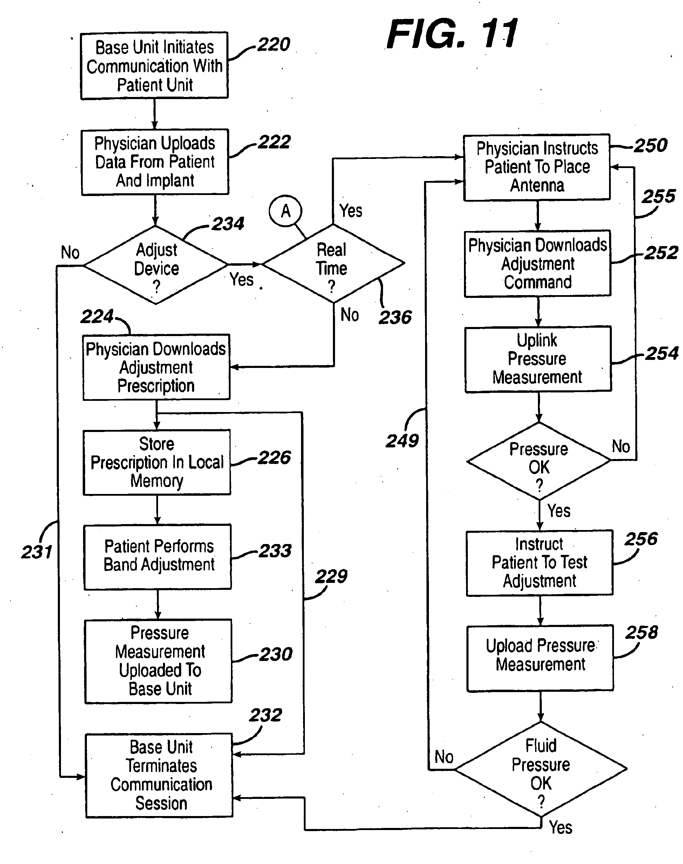

In a second exemplary interaction, shown in FIG. 11, the physician downloads an adjustment prescription for a remotely adjustable device, such as infuser 115 shown in FIG. 6. The physician initiates this communication session through link 180 as shown at step 220. After initiating communications, the physician uploads previously stored data, such as fluid pressure histories, from memory 138 of local unit 60. The physician evaluates the data and determines whether an adjustment is indicated. If the physician chooses an off-line adjustment, an adjustment command is downloaded to local unit 60 and stored in memory 138, as indicated in step 224. With the prescription stored in memory 138, the patient, at his convenience, places antenna 54 over the implant area and initiates the adjustment through local unit 60, as indicated in step 233. Local unit 60 then transmits power and command signals to the implanted microcontroller 106 to execute the adjustment. After the adjustment, the patient establishes a communication link with remote monitoring unit 170 and uploads a series of pressure measurements from the implant to the remote unit. These pressure measurements may be stored in memory 174 of remote unit 170 until accessed by the physician.

In an alternative scenario, the patient may perform a real-time adjustment during a virtual treatment session with the physician. In this situation, the physician establishes communication with the patient through link 180. Once connected through link 180, the physician instructs the patient to place antenna 54 over the implant area, as shown at step 250. After antenna 54 is in position, the physician downloads an adjustment command to infuser 115 through link 180, as shown at step 252. During and/or after the adjustment is executed in infuser 115, a series of pressure measurements are uplinked from infuser 115 to the physician through link 180, as shown at step 254. The physician performs an immediate review of the fluid pressure changes resulting from the adjustment. If the resulting fluid pressure levels are too high or too low, the physician may immediately readjust the restriction band, as indicated by line 255. The physician may also instruct the patient to perform a particular action to test the adjustment, such as drinking or eating, as shown at step 256. As the patient performs the test, the physician may upload pressure measurements from the pressure sensor, as shown at step 258, to evaluate the peristaltic pressure against the band as the patient attempts to pass food or liquid through the stoma. If the pressure measurements are too high, indicating a possible obstruction, the physician may immediately transmit additional command signals to readjust the band and relieve the obstruction, as indicated by line 259. After the physician is satisfied with the results of the adjustment, the communication session is terminated at step 232. In the present invention, local unit 60 is at all times a slave to remote unit 170 so that only a physician can prescribe adjustments, and the patient is prevented from independently executing adjustments through local unit 60.

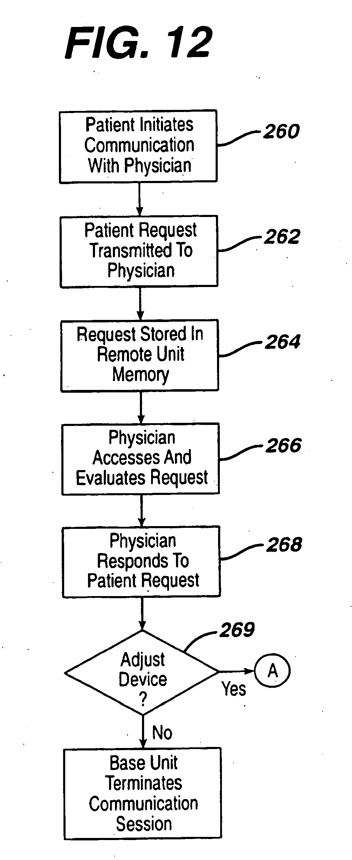

In a third exemplary communication session, shown in FIG. 12, a patient may initiate an interaction with remote unit 170 by entering a request through user interface 140, as shown at step 260. This request may be in the form of an e-mail or other electronic message. At step 262, the patient's request is transmitted through communication link 180 to remote unit 170. At remote unit 170, the patient's request is stored in memory 174 until retrieved at the physician's convenience (step 264). After the physician has reviewed the patient's request (step 266), instructions may be entered through user interface 176 and downloaded to local unit 60. The physician may communicate with the patient regarding treatment or the decision to execute or deny a particular adjustment request, as shown at step 268. If the physician determines at step 269 that an adjustment is required, the physician may initiate a communication session similar to those shown in the flow diagrams of FIGS. 10 and 11. If an adjustment is not indicated, the base unit terminates the session following the responsive communication of step 268.

In addition to the above scenarios, a physician may access local unit 60 at any time to check on patient compliance with previous adjustment instructions, or to remind the patient to perform an adjustment. In these interactions, the physician may contact local unit 60 to request a data upload from memory 138, or transmit a reminder to be stored in memory 138 and displayed the next time the patient turns on local unit 60. Additionally, local unit 60 can include an alarm feature to remind the patient to perform regularly scheduled adjustments, such as diurnal relaxations.

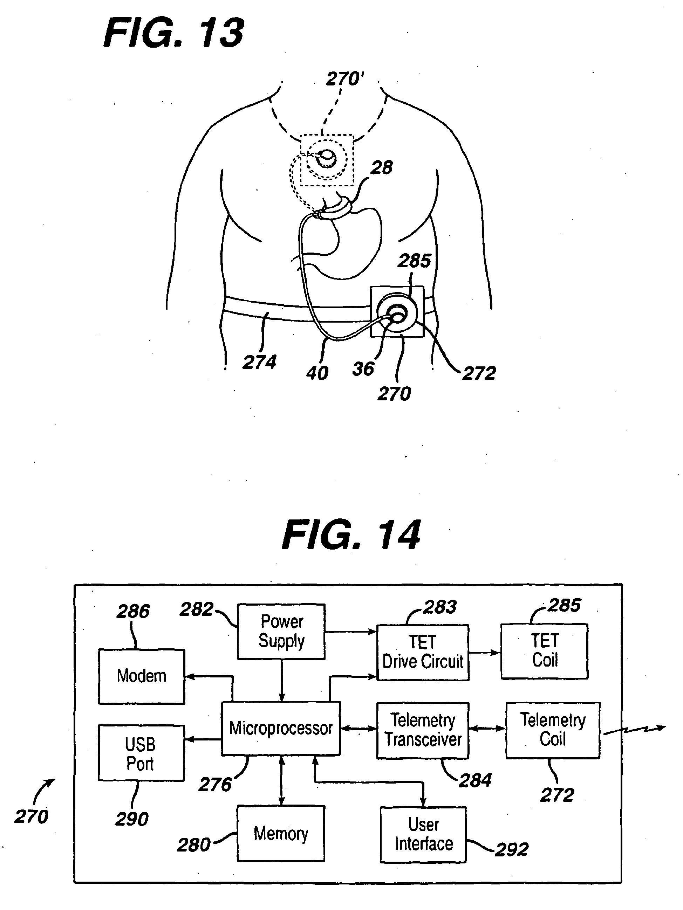

As mentioned above, communication system 20 can be used to uplink a fluid pressure history to remote unit 170 to allow the physician to evaluate the performance of device 22 over a designated time period. FIG. 13 illustrates a data logger 270 that may be used in conjunction with communication system 22 of the present invention to record fluid pressure measurements over a period of time. As shown in FIG. 13, data logger 270 comprises TET and telemetry coils 285, 272 which may be worn by the patient so as to lie adjacent to implanted portion 24. TET coil 285 provides power to the implant, while telemetry coil 272 interrogates the implant and receives data signals, including fluid pressure measurements, through secondary telemetry coil 114. The fluid pressure within the restriction band is repeatedly sensed and transmitted to data logger 270 at an update rate sufficient to measure peristaltic pulses against the band. Typically, this update rate is in the range of 10-20 pressure measurements per second. As shown in FIG. 13, data logger 270 may be worn on a belt 274 about the patient's waist to position coils 272 adjacent injection port 36 when the port is implanted in the patient's abdominal area. Alternatively, data logger 270 can be worn about the patient's neck, as shown by device 270', when injection port 36 is implanted on the patient's sternum. Data logger 270 is worn during waking periods to record fluid pressure variations during the patient's meals and daily routines. At the end of the day, or another set time period, data logger 270 may be removed and the recorded fluid pressure data downloaded to memory 138 of local unit 60. The fluid pressure history may be uploaded from memory 138 to remote unit 170 during a subsequent communication session. Alternatively, fluid pressure data may be directly uploaded from data logger 270 to remote unit 170 using communication link 180.

FIG. 14 shows data logger 270 in greater detail. As shown in FIG. 14, data logger 270 includes a microprocessor 276 for controlling telemetry communications with implanted device 24. Microprocessor 276 is connected to a memory 280 for, among other functions, storing pressure measurements from device 24. While logger 270 is operational, fluid pressure is read and stored in memory 280 at a designated data rate controlled by microprocessor 276. Microprocessor 276 is energized by a power supply 282. To record fluid pressure, microprocessor 276 initially transmits a power signal to implanted portion 24 via TET drive circuit 283 and TET coil 285. After the power signal, microprocessor 276 transmits an interrogation signal to implanted portion 24 via telemetry transceiver 284 and telemetry coil 272. The interrogation signal is intercepted by telemetry coil 114 and transmitted to microcontroller 106. Microcontroller 106 sends a responsive, temperature-adjusted pressure reading from sensor 84 via transceiver 158 and secondary telemetry coil 114. The pressure reading is received through coil 272 and directed by transceiver 284 to microprocessor 276. Microprocessor 276 subsequently stores the pressure measurement and initiates the next interrogation request.

When the patient is finished measuring and recording fluid pressure, logger 270 is removed and the recorded pressure data downloaded to local unit 60, or directly to remote unit 170. As shown in FIGS. 9 and 14, data logger 270 may comprise a modem 286 for transmitting the sensed fluid pressure directly to remote unit 170 using a telephone line 288. The patient may connect logger modem 286 to a telephone line, dial the physician's modem, and select a "send" button on user interface 292. Once connected, microprocessor 276 transmits the stored pressure history through the phone line to microprocessor 172 in remote unit 170. Alternatively, data logger 270 may include a USB port 290 for connecting the logger to local unit 60. Logger USB port 290 may be connected to a USB port 198 on local unit 60 (shown in FIG. 8), and the "send" switch activated to download pressure data to memory 138 in the local unit. After the pressure data is downloaded, logger 270 may be turned off through user interface 292, or reset and placed back on the patient's body for continued pressure measurement.

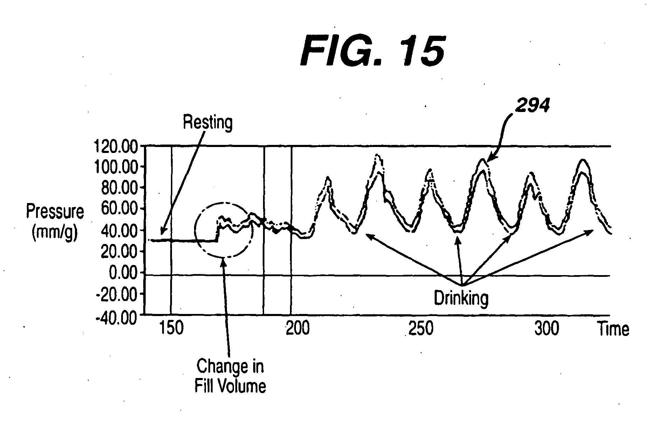

FIG. 15 is a graphical representation of an exemplary pressure signal 294 as measured by sensor 84 during repeated interrogation by local unit 60 or data logger 270 over a sampling time period. Pressure signal 294 may be displayed using graphical user interface 140 of local unit 60 or graphical user interface 176 of remote unit 170. In the example shown in FIG. 15, the fluid pressure in band 28 is initially measured while the patient is stable, resulting in a steady pressure reading as shown. Next, an adjustment is applied to band 28 to decrease the stoma size. During the band adjustment, pressure sensor 84 continues to measure the fluid pressure and transmit the pressure readings through the patient's skin to local unit 60. As seen in the graph of FIG. 15, fluid pressure rises following the band adjustment.

In the example shown, the patient is asked to drink a liquid after the adjustment to check the accuracy of the adjustment. As the patient drinks, pressure sensor 84 continues to measure the pressure spikes due to the peristaltic pressure of swallowing the liquid. The physician may evaluate these pressure spikes from a remote location in order to evaluate and direct the patient's treatment. If the graph indicates pressure spikes exceeding desired levels, the physician may immediately take corrective action through communication system 20, and view the results of the corrective action, until the desired results are achieved. Accordingly, through communication system 20 a physician can perform an adjustment and visually see the results of the adjustment, even when located at a considerable distance from the patient.

In addition to adjustments, communication system 20 can be used to track the performance of an intake restriction device over a period of time. In particular, a sampling of pressure measurements from data logger 270 may be uploaded to the physician's office for evaluation. The physician may visually check a graph of the pressure readings to evaluate the performance of the restriction device. Pressure measurement logs can be regularly transmitted to remote monitoring unit 170 to provide a physician with a diagnostic tool to ensure that a food intake restriction device is operating effectively. If any abnormalities appear, the physician may use communication system 20 to contact the patient and request additional physiological data or prescribe an adjustment. In particular, communication system 20 may be utilized to detect a no pressure condition within band 28, indicating a fluid leakage. Alternatively, system 20 may be used to detect excessive pressure spikes within band 28, indicating a kink in catheter 40 or a blockage within the stoma. Using local unit 60, the patient can also evaluate pressure readings at home and notify their physician when the band pressure drops below a specified baseline, indicating the need for an adjustment of the device. Communication system 20 thus has benefits as a diagnostic and monitoring tool during patient treatment with a bariatric device. The convenience of evaluating an intake restriction device 22 through communication system 20 facilitates more frequent monitoring and adjustments of the device.

It will become readily apparent to those skilled in the art that the above invention has equally applicability to other types of implantable bands. For example, bands are used for the treatment of fecal incontinence. One such band is described in U.S. Patent 6,461,292 which is hereby incorporated herein by reference. Bands can also be used to treat urinary incontinence. One such band is described in U.S. Patent Application 2003/0105385. Bands can also be used to treat heartburn and/or acid reflux. One such band is described in U.S. Patent 6,470,892 . Bands can also be used to treat impotence. One such band is described in U.S. Patent Application 2003/0114729 .

While preferred embodiments of the present invention have been shown and described herein, it will be obvious to those skilled in the art that such embodiments are provided by way of example only. Numerous variations, changes, and substitutions will now occur to those skilled in the art without departing from the invention. For example, as would be apparent to those skilled in the art, the disclosures herein have equal application in robotic-assisted surgery. In addition, it should be understood that every structure described above has a function and such structure can be referred to as a means for performing that function. Accordingly, it is intended that the invention be limited only by the scope of the appended claims.

While the present invention has been illustrated by description of several embodiments, numerous other variations, changes, and substitutions will occur to those skilled in the art without departing from the scope of the invention. For instance, the device and method of the present invention has been illustrated with respect to transmitting pressure data from the implant to the remote monitoring unit. However, other types of data may also be transmitted to enable a physician to monitor a plurality of different aspects of the restrictive opening implant. Additionally, the present invention is described with respect to a food intake restriction device for bariatric treatment. The present invention is not limited to this application, and may also be utilized with other restrictive opening implants or artificial sphincters without departing from the scope of the invention. The structure of each element associated with the present invention can be alternatively described as a means for providing the function performed by the element. It will be understood that the foregoing description is provided by way of example, and that other modifications may occur to those skilled in the art without departing from the scope of the appended Claims.

## Claims

1. A bi-directional communication system for use with a restrictive opening device (28) implanted within a patient, the system comprising:
a sensor means (84) for measuring fluid pressure within the restrictive opening device (28);
b. means (114) for communicating fluid pressure measurements from the sensor means (84) to a local unit (60) external to the patient;
c. a base unit (170) at a remote location from the patient, the base unit (170) including user interface means for evaluating the fluid pressure measurements;
d. a communication link between the local and base units (60, 170) for transmitting data between the units, the transmitted data including the fluid pressure measurements; and **characterized in that:**
the base unit (170) includes means for determining the size of a stoma formed by the restrictive opening device (28).

2. The bi-directional communication system of claim 1, wherein the user interface means further comprises means for entering an adjustment command for the restrictive opening device (28).

3. The bi-directional communication system of claim 2, wherein the adjustment command is transmitted between the base and local units (170, 60) through the communication link.

4. The bi-directional communication system of claim 3, wherein the communication link comprises an Internet connection between the local and base units (60, 170).

5. The bo-directional communication system of claim 3, wherein the communication link comprises a telephone network.

6. The bi-directional communication system of claim 1, wherein the communicating means further comprises a portable data recording device (270) capable of being worn by the patient for recording fluid pressure measurements from the restrictive opening device (28) over a sampling time period.

7. The bi-directional communication system of claim 6, further comprising means for transmitting fluid pressure measurements directly from the portable data recording device (270) to the base unit (170) through a communication link.

8. The bi-directional communication system of claim 3, further comprising:
a. means for transmitting the adjustment command to the restrictive opening device (28); and
b. a control means in the restrictive opening device (28) for adjusting the device in response to the adjustment command.

9. A method for communicating data between a restrictive opening device (28) implanted in a patient, and a base unit (170) remotely located from the patient, the method comprising the steps of:
a. measuring fluid pressure in the restrictive opening device (28);
b. retrieving fluid pressure measurements from the restrictive opening device (28);
c. transmitting the retrieved fluid pressure measurements to the base unit (170); and **characterized by**:
d. evaluating the fluid pressure measurements at the base unit (170) to determine the size of a stoma formed by the restrictive opening device (28).

## Patentansprüche

1. Bidirektionales Kommunikationssystem zur Verwendung mit einem eine Öffnung beschränkenden Element (28), das in einen Patienten implantiert ist, wobei das System aufwei st:
a. eine Sensoreinrichtung (84) zum Messen des Fluiddruckes innerhalb des eine Öffnung beschränkenden Elements (28);
b. eine Einrichtung (114) zum Übermitteln von Fluiddruckmessungen von der Sensorcinrichtung (84) zu einer lokalen Einheit (60) außerhalb des Patienten;
c. eine Basiseinheit (170) an einem Ort entfernt von dem Patienten, wobei die Basiseinheit (170) eine Benutzerschnittstelleneinrichtung zum Bewerten der Fluiddruckmessungen umfasst;
d. eine Kommunikationsverbindung zwischen der lokalen Einheit und der Basiseinheit (60, 170) zum Senden von Daten zwischen den Einheiten, wobei die gesendeten Daten die Fluiddruckmessungen umfassen; und **dadurch gekennzeichnet ist, dass**:
die Basiseinheit (170) eine Einrichtung zum Bestimmen der Größe einer Körperöffnung, die durch das eine Öffnung beschränkende Element (28) gebildet ist, umfasst.

2. Bidirektionales Kommunikationssystem nach Anspruch 1, bei dem die Benutzerschnittstelleneinrichtung weiter eine Einrichtung zum Eingeben eines Einstellbefehls für das eine Öffnung beschränkende Element (28) aufweist.

3. Bidirektionales Kommunikationssystem nach Anspruch 2, bei dem der Einstellbefehl zwischen der Basiseinheit und der lokalen Einheit (170, 60) durch die Kommunikationsverbindung gesendet wird.

4. Bidirektionales Kommunikationssystem nach Anspruch 3, bei dem die Kommunikationsverbindung eine Internet-Verbindung zwischen der lokalen Einheit und der Basiseinheit (60, 170) aufweiset.

5. Bidirektionales Kommunikationssystem nach Anspruch 3, bei dem die Kommunikationsverbindung ein Telefonnetzwerk aufweist.

6. Bidirektionales Kommunikationssystem nach Anspruch 1, bei dem die Kommunikationseinirichtung weiter ein tragbares Datenaufzeichnungsgerät (270) aufweist, das von dem Patienten getragen werden kann, zum Aufzeichnen von Fluiddruckmessungen von dem eine Öffnung beschränkenden Element (28) über eine Abtastzeitdauer.

7. Bidirektionales Kommunikationssystem nach Anspruch 6, weiter mit einer Einrichtung zum Senden von Fluiddruckmessungen direkt von dem tragbaren Datenaufzeichnungsgerät (270) zu der Basiseinheit (170) durch eine Kommunikationsverbindung.

8. Bidirektionales Kommunikationssystem nach Anspruch 3, das weiter aufweist:
a. eine Einrichtung zum Senden des Einstellbefehls an das eine Öffnung beschränkende Element (28); und
b. eine Steuereinrichtung in dem eine Öffnung beschränkenden Element (28) zum Einstellen des Elementes als Antwort auf den Einstellbefehl.

9. Verfahren zum Kommunizieren von Daten zwischen einem eine Öffnung beschränkenden Element (28), das in einen Patienten implantiert ist, und einer Basiseinheit (170), die sich entfernt von dem Patienten befindet, wobei das Verfahren die Schritte aufweist:
a. Messen des Fluiddruckes in dem eine Öffnung beschränkenden Element (28);
b. Gewinnen von Fluiddruckmessungen von dem eine Öffnung beschränkenden Element (28);
c. Senden der gewonnenen Fluiddruckmessungen an die Basiseinheit (170); und **gekennzeichnet durch**:
d. Bewerten der Fluiddruckmessungen an der Basiseinheit (170), um die Größe einer Körperöffnung zu bestimmen, die **durch** das eine Öffnung beschränkende Element (28) gebildet wird.

## Revendications

1. Système de communication bidirectionnel à utiliser avec un dispositif d'ouverture restric tif (28) implanté dans un patient, le système comprenant :
a. des systèmes de capteur (84) pour mesurer la pression de fluide dans le dispositif d'ouverture restrictif (28) ;
b. des moyens (114) pour communiquer les mesures de pression de fluide provenant des systèmes de capteur (84) à une unité locale (60) extérieure au patient ;
c. une unité de base (170) à un endroit éloigné du patient, l'unité de base (170) comprenant des systèmes d'interface utilisateur, afin d'évaluer les mesures de pression de fluide ;
d. une liaison de communication entre les unités locale et de base (60, 170), pour transmettre des données entre les unités, les données transmises comprenant les mesures de pression de fluide ; et **caractérisée en ce que :**
· l'unité de base (170) comprend des moyens pour déterminer la taille d'un stoma formé par le dispositif d'ouverture restrictif (28).

2. Système de communication bidirectionnel selon la revendication 1, dans lequel le système d'interface utilisateur comprend en outre des moyens pour entrer une commande der réglage pour le dispositif d'ouverture restrictif (28).

3. Système de communication bidirectionnel selon la revendication 2, dans lequel la commande de réglage est transmise entre les unités de base et locale (170, 60) à travers la liaison de communication.

4. Système de communication bidirectionnel selon la revendication 3, dans lequel la liaison de communication comprend une connexion Internet entre les unités locale et de base (60, 170).

5. Système do communication bidirectionnel selon la revendication 3, dans lequel la liaison de communication comprend un réseau téléphonique.

6. Système de communication bidirectionnel selon la revendication 1, dans lequel le système de communication comprend en outre un dispositif d'enregistrement de données portable (270), capable d'être porté par le patient pour enregistrer des mesures de pression de fluide provenant du dispositif d'ouverture restrictif (28) sur une période d'échantillonnage.

7. Système de communication bidirectionnel selon la revendication 6, comprenant en outre des moyens pour transmettre des mesures de pression de fluide directement depuis le dispositif d'enregistrement de données portable (270) vers l'unité de base (170) à travers une liaison de communication.

8. Système de communication bidirectionnel selon la revendication 3, comprenant en outre :
a. un moyen de transmission de la commande de réglage au dispositif d'ouverture restrictif (28) ; et
b. un moyen de contrôle dans le dispositif d'ouverture restrictif (28) pour ajuster le dispositif en réponse à la commande de réglage.

9. Procédé de communication de données entre un dispositif d'ouverture restrictif (28) implanté dans un patient, et une unité de base (170) éloignée du patient, le procédé comprenant les étapes consistant à :
a. mesurer la pression de fluide dans le dispositif d'ouverture restrictif (28) ;
b. récupérer des mesures de pression de fluide du dispositif d'ouverture restrictif (28) ;
c. transmettre les mesures de pression de fluide récupérées à l'unité de base (170) et **caractérisé par** :
d. l'évaluation des mesures de pression de fluide à à l'unité de base (170) afin de déterminer la taille d'un stoma formé par le dispositif d'ouverture restrictif (28).
